# FASCICULE DE BREVET EUROPEEN

(11) **EP 3 797 002 B1**
(45) Date de publication et mention de la délivrance du brevet: **06.07.2022**
(21) Numéro de dépôt: 19737163.6
(22) Date de dépôt: 20.05.2019
(51) Int. Cl.: B05B 11/00, B05B 11/06, A61M 11/00, A61M 15/00, A61M 15/08, B05B 15/55, B65D 83/66, B65D 83/68

(54) **DISPOSITIF DE DISTRIBUTION DE PRODUIT FLUIDE**
FLÜSSIGPRODUKTSPENDER
FLUID PRODUCT DISPENSER

(30) Priorité: 23.05.2018 FR 1854278
(43) Date de publication de la demande: 31.03.2021
(73) Titulaire: APTAR France SAS, 27110 Le Neubourg (FR)
(72) Inventeur: PETIT, Ludovic, 27110 VITOT (FR)
(74) Mandataire: CAPRI
(86) Numéro de dépôt international: PCT/FR2019/051137
(87) Numéro de publication internationale: WO 2019/224462

(56) Documents cités:
- EP-A1- 0 557 714
- WO-A1-02/00524

## Description

La présente invention concerne un dispositif de distribution de produit fluide.

Les dispositifs de distribution de produit fluide sont bien connus. Ils comportent généralement un réservoir contenant une ou plusieurs doses de produit fluide, sous forme de liquide, de gel, de mousse ou de produit fluide, des moyens de distribution, tels qu'une pompe, une valve ou une chasse d'air, et une tête de distribution comportant un orifice de distribution. Lorsque le dispositif de distribution est actionné, une dose de produit fluide est distribuée, par exemple dans une narine de l'utilisateur.

Un inconvénient avec ces dispositifs de l'art antérieur concerne notamment l'amorçage de la pompe, qui nécessite un ou plusieurs actionnements à vide ou à dose partielle avant de pouvoir distribuer une dose complète. Ce problème se pose à chaque fois que l'utilisateur souhaite actionner le dispositif, en particulier lorsqu'un certain laps de temps s'est écoulé depuis le dernier actionnement.

Les documents EP0557714 et WO0200524 décrivent des dispositifs de l'état de la technique.

La présente invention a pour but de fournir un dispositif de distribution de produit fluide qui ne reproduit pas les inconvénients susmentionnés.

La présente invention a également pour but de fournir un tel dispositif qui est simple et peu coûteux à fabriquer et à assembler.

La présente a donc pour objet un dispositif de distribution nasale de produit fluide comportant:
- un corps externe pourvu d'un orifice de distribution,
- un corps interne, mobile axialement par rapport audit corps externe entre une position de repos et une position d'actionnement, ledit corps interne formant réservoir contenant le produit fluide à distribuer,
- un premier élément élastique, tel qu'un ressort, pour solliciter ledit corps interne vers sa position de repos,
- un organe tubulaire axial fixé audit corps externe, à l'intérieur dudit corps interne, et définissant en partie un canal d'expulsion se terminant par ledit orifice de distribution,
- un piston monté coulissant dans ledit réservoir, autour dudit organe tubulaire axial, entre une position de repos et une position de distribution,
- un second élément élastique, tel qu'un ressort, pour solliciter ledit piston vers sa position de repos,
- une chambre de dosage définie dans ledit corps interne entre ledit piston et ledit organe tubulaire axial, ladite chambre de dosage étant, en position de repos, reliée audit réservoir et isolée dudit canal d'expulsion, et étant, en cours d'actionnement, isolée dudit réservoir et reliée audit canal d'expulsion,
- ladite chambre de dosage étant, en position droite d'utilisation et de stockage, disposée sous ledit réservoir, pour ainsi au repos se remplir automatiquement par gravité avec le produit fluide contenu dans ledit réservoir.

Avantageusement, dans sa position de repos, ledit piston obture au moins un canal radial dudit organe tubulaire axial, pour isoler ladite chambre de dosage dudit canal d'expulsion.

Avantageusement, lorsque le dispositif est actionné, ledit piston est déplacé axialement autour dudit organe tubulaire axial vers sa position de distribution, pour ainsi relier ladite chambre de dosage audit canal d'expulsion via ledit au moins un canal radial.

Avantageusement, ladite chambre de dosage s'étend autour et sous une extrémité distale dudit organe tubulaire axial.

Avantageusement, ladite partie de la chambre de dosage disposée sous ledit organe tubulaire axial est formée par un puits réalisé dans un fond axial dudit corps interne.

Avantageusement, une projection radiale externe dudit organe tubulaire axial coopère de manière étanche avec ledit corps interne, au repos et lors de l'actionnement, pour isoler le réservoir de l'atmosphère.

Avantageusement, ledit réservoir comporte une partie de plus grand diamètre, coopérant avec ledit piston en position de repos, et une partie de plus petit diamètre, coopérant avec ledit piston lors de l'actionnement.

Avantageusement, ledit piston comporte une lèvre externe pour réaliser, en cours d'actionnement, l'étanchéité avec la partie de plus petit diamètre, ladite partie de plus grand diamètre comportant des nervures pour garantir, en position de repos, un passage de fluide entre ledit réservoir et ledit piston vers ladite chambre de dosage.

Avantageusement, le dispositif comporte en outre un insert disposé dans ledit canal d'expulsion pour limiter le volume mort dudit canal d'expulsion.

Avantageusement, ledit dispositif de distribution comporte en outre un système de génération d'écoulement de gaz comprimé adapté à distribuer un écoulement de gaz comprimé à chaque actionnement.

Avantageusement, ledit système de génération d'écoulement de gaz comprimé comprend un second réservoir contenant une pluralité de doses d'au moins un gaz propulseur pressurisé et/ou liquéfié, une valve doseuse étant montée sur ledit second réservoir pour distribuer une dose de gaz propulseur à chaque actionnement dudit ensemble de distribution.

Avantageusement, ladite valve doseuse comportant une soupape déplaçable axialement et qui s'étend à l'intérieur de ladite chambre de dosage, de sorte qu'en fin d'actionnement, ledit organe tubulaire axial vient pousser axialement ladite soupape et ainsi actionner la valve, provoquant l'expulsion d'un écoulement de gaz comprimé qui va pousser la dose de produit fluide à travers l'orifice de distribution et/ou purger le canal de distribution de tout produit résiduel.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section transversale d'un dispositif de distribution de produit fluide selon un premier mode de réalisation avantageux, en position de repos,
- la figure 2 est une vue du détail A1 de la figure 1,
- la figure 3 est une vue similaire à celle de la figure 1, en cours d'actionnement,
- la figure 4 est une vue du détail A3 de la figure 3,
- la figure 5 est une vue similaire à celles des figures 1 et 3, en fin d'actionnement,
- la figure 6 est une vue du détail A5 de la figure 5,
- la figure 7 est une vue schématique en section transversale d'un dispositif de distribution de produit fluide selon un second mode de réalisation avantageux, en position de repos,
- la figure 8 est une vue du détail A7 de la figure 7,
- la figure 9 est une vue schématique en section transversale d'un dispositif de distribution de produit fluide selon un troisième mode de réalisation avantageux, en position de repos,
- la figure 10 est une vue du détail A9 de la figure 9,
- la figure 11 est une vue similaire à celle de la figure 10, en début d'actionnement,
- la figure 12 est une vue du détail A11 de la figure 11,
- la figure 13 est une vue similaire à celles des figures 9 et 11, en cours d'actionnement,
- la figure 14 est une vue du détail A13 de la figure 13, et
- la figure 15 est une vue similaire à celles des figure 9, 11 et 13, en fin d'actionnement.

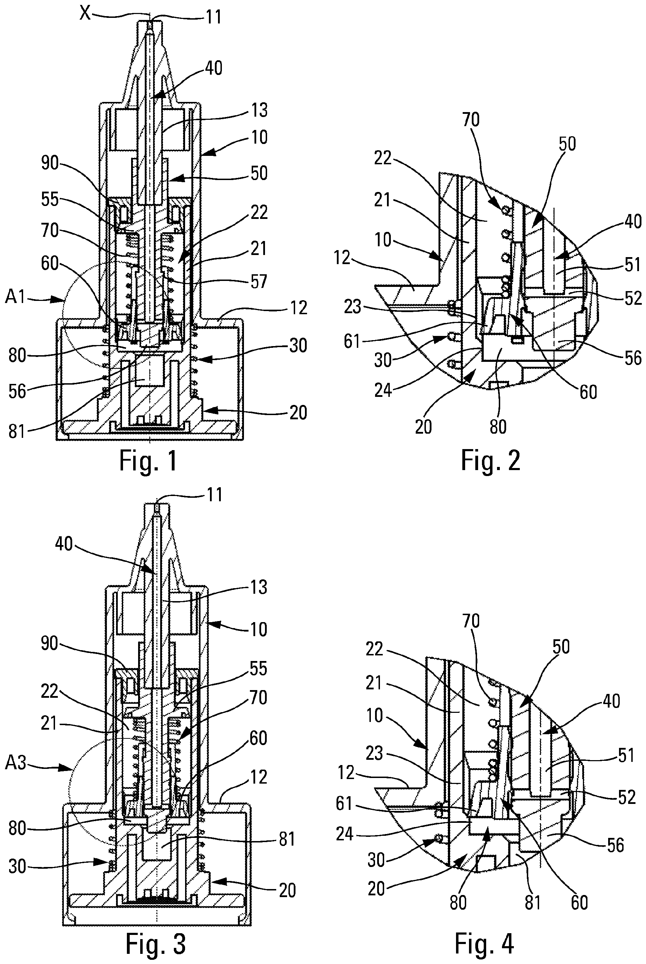

Dans la description, les termes "axial" et "radial" se réfèrent à l'axe longitudinal X représenté notamment sur la figure 1. Les termes "proximal" et "distal" se réfèrent à l'orifice de distribution. Les termes "aval" et "amont" se réfèrent au sens d'écoulement du produit fluide lors de sa distribution.

Les figures 1 à 6 représentent un premier mode de réalisation avantageux.

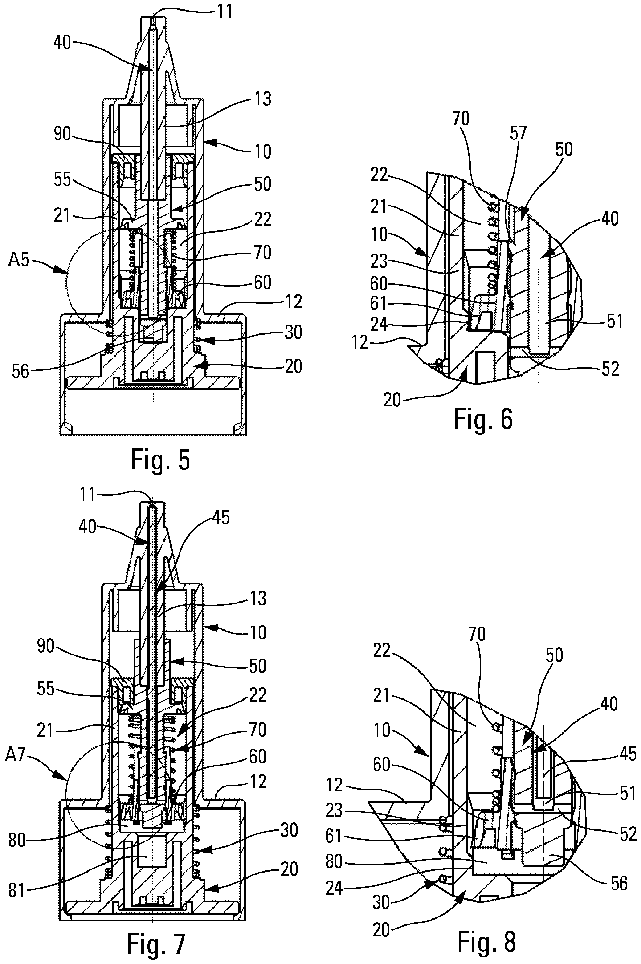

Le dispositif de distribution de produit fluide, de préférence un dispositif de distribution nasale, comporte un corps externe 10 et un corps interne 20, mobile axialement par rapport audit corps externe 10 entre une position de repos, représentée sur les figures 1 et 2, et une position d'actionnement, représentée sur les figures 5 et 6. Un premier élément élastique 30, tel qu'un ressort, sollicite ledit corps interne 20 vers sa position de repos.

Le corps externe 10 forme une tête de distribution, pourvue d'un orifice de distribution 11, orienté de préférence axialement. Le corps externe 10 peut également comporter une bride radiale externe 12 formant repose-doigts, pour l'actionnement du dispositif.

Le corps interne 20 comporte un manchon creux 21 formant réservoir 22 contenant le produit fluide à distribuer.

L'orifice de distribution 11 est disposé à l'extrémité aval d'un canal d'expulsion 40. Dans l'exemple de la figure 1, ce canal d'expulsion 40 est formé en partie d'une extension tubulaire axiale interne 13 dudit corps externe 10, et en partie d'un organe tubulaire axial 50 fixée à ladite extension tubulaire interne 13. D'autres mises en oeuvre sont envisageables.

Ledit organe tubulaire axial 50 comporte un canal axial central 51, relié à ladite extension tubulaire axiale interne 13 dudit corps externe 10, et au moins un canal radial 52, disposé à proximité de l'extrémité distale dudit organe tubulaire axial 50 et relié audit réservoir 22 lorsque le dispositif est actionné. Ledit canal axial central 51 et ledit au moins un canal radial 52 forment donc également partie du canal d'expulsion 40.

Un piston 60 est monté coulissant dans ledit manchon creux 21 formant réservoir 22, autour dudit organe tubulaire axial 50, entre une position de repos et une position de distribution. Un second élément élastique 70, tel qu'un ressort, sollicite ledit piston 60 vers sa position de repos. Avantageusement, ledit second élément élastique 70 coopère d'une part avec ledit piston 60 et d'autre part avec une projection radiale externe 55 dudit organe tubulaire axial 50.

Ledit piston 60 définit une chambre de dosage 80, qui, en position de repos du piston 60, est reliée audit réservoir 22, et qui, en cours d'actionnement, lorsque le piston 60 est déplacé vers sa position de distribution, est isolée dudit réservoir 22, pour définir une dose précise de produit fluide à distribuer à chaque actionnement.

Comme visible sur les figures 1 à 6, ladite chambre de dosage 80 est disposée sous ledit réservoir 22, dans la position droite du dispositif représentée sur les figures, qui est à la fois la position de stockage du dispositif et sa position d'utilisation. Ainsi, au repos, la chambre de dosage 80 se remplit automatiquement par gravité avec le produit fluide contenu dans le réservoir 22.

La paroi cylindrique interne dudit manchon creux 21 formant réservoir 22 comporte une partie de plus grand diamètre 23, coopérant avec ledit piston 60 en position de repos, et une partie de plus petit diamètre 24, coopérant avec ledit piston 60 lors de l'actionnement. Avantageusement, ledit piston 60 a une lèvre externe 61 pour réaliser l'étanchéité avec la partie de plus petit diamètre 24, en cours d'actionnement. La partie de plus grand diamètre 23 comporte avantageusement des nervures pour garantir un passage de fluide entre le réservoir 22 et le piston 60 vers la chambre de dosage 80 en position de repos.

Dans sa position de repos, ledit piston 60 obture ledit au moins un canal radial 52 dudit organe tubulaire axial 50, pour isoler ladite chambre de dosage 80 dudit canal d'expulsion 40. Lorsque le dispositif est actionné, le piston 60 est déplacé axialement autour dudit organe tubulaire axial 50 vers sa position de distribution, pour ainsi relier ladite chambre de dosage 80 audit canal d'expulsion 40 via ledit au moins un canal radial 52, pour permettre l'expulsion d'une dose de produit fluide.

Ainsi, au repos, la chambre de dosage 80 est reliée au réservoir 22 et isolée du canal d'expulsion 40, et en cours d'actionnement, la chambre de dosage 80 est isolée dudit réservoir 22 et reliée audit canal d'expulsion 40.

Avantageusement, la chambre de dosage 80 au repos s'étend à la fois autour et sous l'extrémité distale 56 dudit organe tubulaire axial 50. Ladite partie de la chambre de dosage 80 disposée sous l'organe tubulaire axial 50 est de préférence formée par un puits 81 réalisé dans un fond axial dudit corps interne 20. Comme visible sur la figure 5, en position de distribution, ladite extrémité axiale distale 56 de l'organe tubulaire axial 50 pénètre dans ledit puits 81, pour ainsi réduire au maximum le volume mort de ladite chambre de dosage 80.

De préférence, l'organe tubulaire axial 50 comporte une butée axiale 57 pour le piston 60, pour définir la position limite du piston 60 par rapport audit organe tubulaire axial 50 en position de distribution.

Avantageusement, ladite projection radiale externe 55 dudit organe tubulaire axial 50 coopère de manière étanche avec ledit corps interne 20, au repos et lors de l'actionnement, pour isoler le réservoir 22 de l'atmosphère. Dans l'exemple représenté, on prévoit en outre une virole 90 fixée sur l'extrémité axiale proximale dudit corps interne 20, autour dudit organe tubulaire axial 50, notamment pour renforcer l'étanchéité.

Les figures 7 et 8 illustrent un second mode de réalisation, très similaire à celui décrit ci-dessus, dans lequel le dispositif comporte en outre un insert 45 dans le canal d'expulsion 40. Cet insert 45, également appelé gicleur interne, permet de limiter le volume mort dudit canal d'expulsion 40, et éventuellement de former un profil de pulvérisation juste en amont de l'orifice de distribution 11. Il peut être notamment formé d'une tige emmanchée dans ledit canal d'expulsion 40, et s'étendant depuis ledit orifice de distribution 11 jusqu'aux abords dudit au moins un canal radial 52, comme visible notamment sur la figure 8.

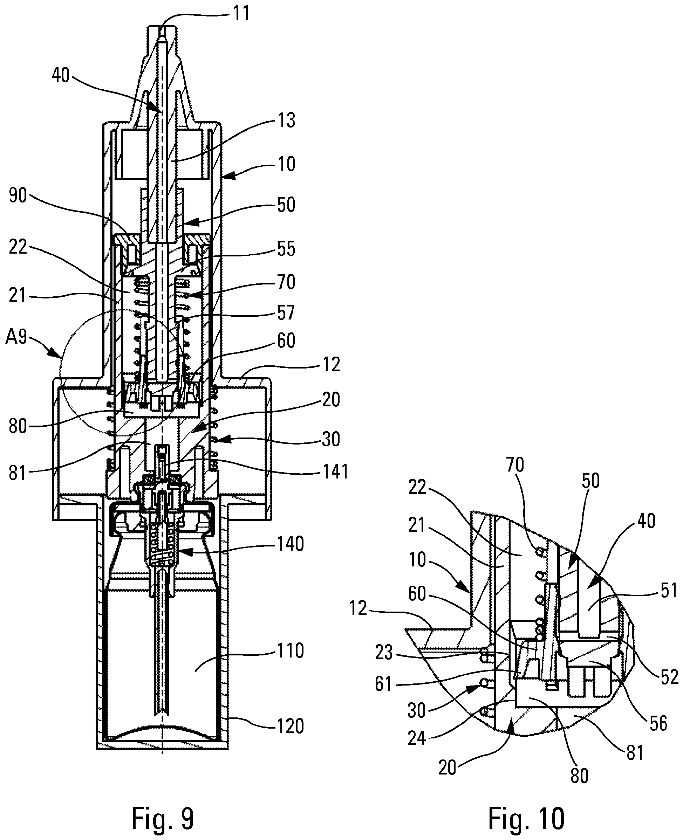

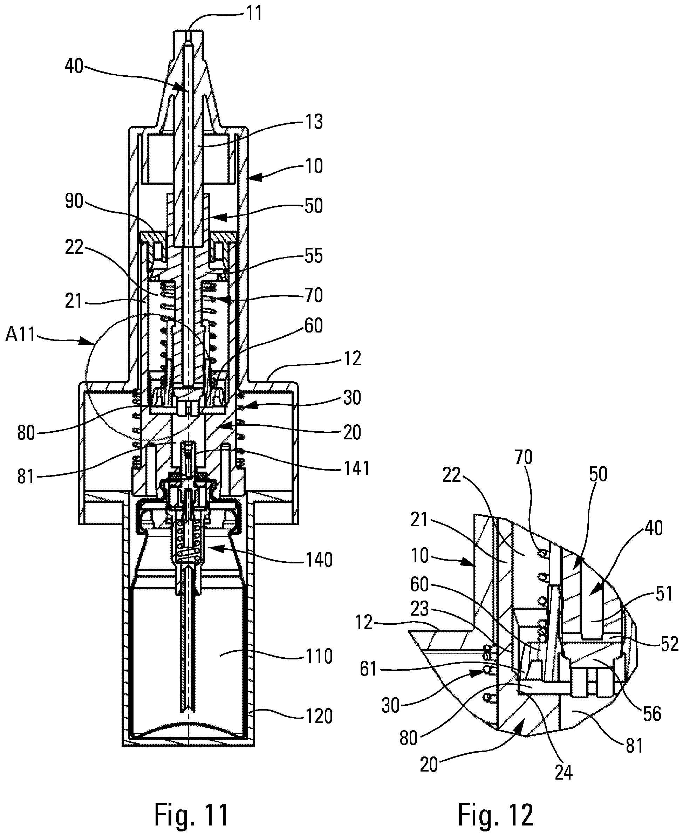

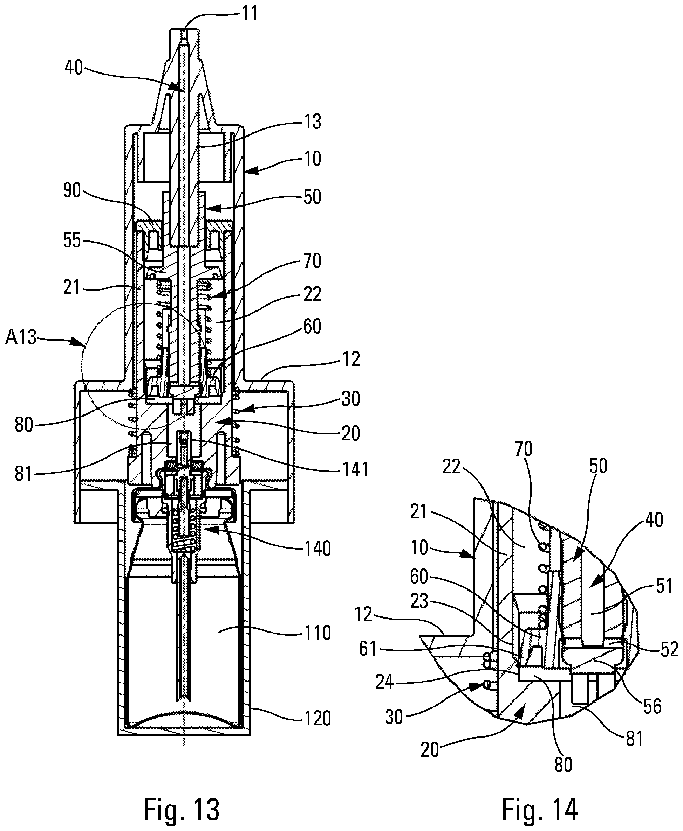

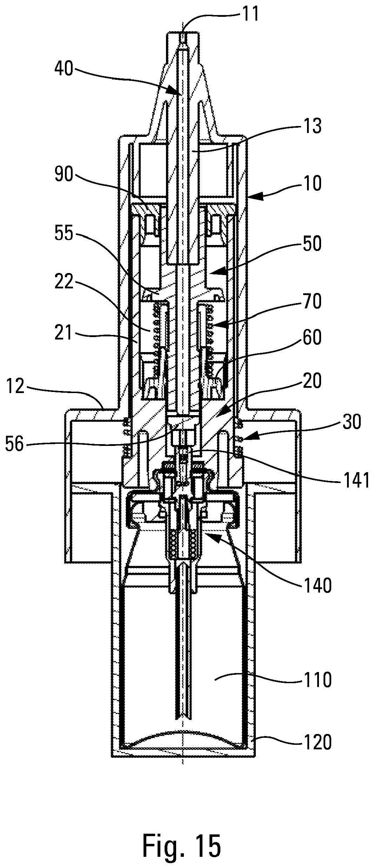

Les figures 9 à 15 illustrent un troisième mode de réalisation avantageux, dans lequel le dispositif de distribution, similaire à celui décrit ci-dessus en référence aux figures 1 à 6, comporte en outre un système de génération d'écoulement de gaz comprimé adapté à distribuer un écoulement de gaz comprimé à chaque actionnement.

Selon un mode de réalisation préféré, ce système de génération d'écoulement de gaz comprimé comprend un second réservoir 110 contenant une pluralité de doses d'au moins un gaz propulseur pressurisé et/ou liquéfié. Avantageusement, ledit second réservoir 110 ne comporte que du gaz propulseur. En variante toutefois, ledit second réservoir 110 peut contenir, outre ledit gaz propulseur, un second produit fluide, adapté à se combiner avec le produit fluide issu du réservoir 22.

Une valve doseuse 140 est montée sur ledit second réservoir 110 pour distribuer une dose de gaz propulseur à chaque actionnement dudit ensemble de distribution. De manière connue, ladite valve doseuse 140 comportant une soupape 141 qui, lors de l'actionnement dudit ensemble de distribution, se déplace axialement le long dudit axe longitudinal X. La valve doseuse 140 peut être montée sur ledit second réservoir 110 au moyen d'une capsule de fixation, par exemple sertissable sur le col dudit second réservoir. La structure de la valve doseuse 140 et sa fixation audit second réservoir 110 peuvent toutefois être réalisées d'une quelconque manière connue, et la présente invention ne se limite pas aux exemples représentés sur les dessins.

Comme visible notamment sur la figure 9, ladite soupape 141 s'étend axialement à l'intérieur dudit puits 81 de la chambre de dosage 80. Ainsi, en fin d'actionnement, l'extrémité axiale distale 56 de l'organe tubulaire axial 50 vient pousser ladite soupape 141 et ainsi actionner la valve 140. Eventuellement, ladite extrémité axiale distale 56 peut être pourvue de tige(s) axiale(s) pour coopérer avec ladite soupape 141.

Avantageusement, le second réservoir 110 est inséré dans un corps inférieur 120, monté coulissant par rapport audit corps externe.

En variante à l'utilisation d'une valve doseuse montée sur un réservoir contenant du gaz propulseur, on pourrait envisager une chasse d'air qui à chaque actionnement comprime de l'air dans une chambre et délivre un écoulement d'air comprimé. Cette chambre d'air peut comporter un clapet d'entrée, par exemple formé par une membrane permettant une reprise d'air après chaque actionnement, et un clapet de sortie, par exemple formé par une bille. D'autres mises en œuvre sont toutefois possibles.

Lors de l'actionnement, l'utilisateur place un ou deux doigts sur le repose-doigts 12 du corps externe 10 et le pouce sous ledit second réservoir 110 (ou sous le corps inférieur 120), et les déplace axialement l'un par rapport l'autre. Ceci va d'abord déplacer le piston 60 et donc actionner la distribution d'une dose de produit fluide, comme décrit précédemment. En fin d'actionnement, l'organe tubulaire axial 50 pénètre dans le puits 81 et vient en contact avec la soupape 141 de la valve 140, pour actionner le système de génération d'écoulement de gaz comprimé. Ceci va provoquer l'expulsion d'un écoulement de gaz comprimé qui va pousser la dose de produit fluide à travers l'orifice de distribution 11 et/ou purger le canal de distribution 40 de tout produit résiduel.

La présente invention a été décrite en référence à des modes de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant :
- un corps externe (10) pourvu d'un orifice de distribution (11),
- un corps interne (20), mobile axialement par rapport audit corps externe (10) entre une position de repos et une position d'actionnement, ledit corps interne (20) formant un réservoir (22) contenant le produit fluide à distribuer,
- un premier élément élastique (30), tel qu'un ressort, pour solliciter ledit corps interne (20) vers sa position de repos,
- un organe tubulaire axial (50) fixé audit corps externe (10), à l'intérieur dudit corps interne (20), et définissant en partie un canal d'expulsion (40) se terminant par ledit orifice de distribution (11),
- un piston (60) monté coulissant dans ledit réservoir (22), autour dudit organe tubulaire axial (50), entre une position de repos et une position de distribution,
- un second élément élastique (70), tel qu'un ressort, pour solliciter ledit piston (60) vers sa position de repos,
le dispositif de distribution étant **caractérisé en ce qu'**il comporte en outre :
- une chambre de dosage (80) définie dans ledit corps interne (20) entre ledit piston (60) et ledit organe tubulaire axial (50), ladite chambre de dosage (80) étant, en position de repos, reliée audit réservoir (22) et isolée dudit canal d'expulsion (40), et étant, en cours d'actionnement, isolée dudit réservoir (22) et reliée audit canal d'expulsion (40),
- ladite chambre de dosage (80) étant, en position droite d'utilisation et de stockage, disposée sous ledit réservoir (22), pour ainsi au repos se remplir automatiquement par gravité avec le produit fluide contenu dans ledit réservoir.

2. Dispositif selon la revendication 1, dans lequel, dans sa position de repos, ledit piston (60) obture au moins un canal radial (52) dudit organe tubulaire axial (50), pour isoler ladite chambre de dosage (80) dudit canal d'expulsion (40).

3. Dispositif selon la revendication 2, dans lequel, lorsque le dispositif est actionné, ledit piston (60) est déplacé axialement autour dudit organe tubulaire axial (50) vers sa position de distribution, pour ainsi relier ladite chambre de dosage (80) audit canal d'expulsion (40) via ledit au moins un canal radial (52).

4. Dispositif selon l'une quelconque des revendications précédentes, dans lequel ladite chambre de dosage (80) s'étend autour et sous une extrémité distale (56) dudit organe tubulaire axial (50).

5. Dispositif selon la revendication 4, dans lequel ladite partie de la chambre de dosage (80) disposée sous ledit organe tubulaire axial (50) est formée par un puits (81) réalisé dans un fond axial dudit corps interne (20).

6. Dispositif selon l'une quelconque des revendications précédentes, dans lequel une projection radiale externe (55) dudit organe tubulaire axial (50) coopère de manière étanche avec ledit corps interne (20), au repos et lors de l'actionnement, pour isoler le réservoir (22) de l'atmosphère.

7. Dispositif selon l'une quelconque des revendications précédentes, dans lequel ledit réservoir (22) comporte une partie de plus grand diamètre (23), coopérant avec ledit piston (60) en position de repos, et une partie de plus petit diamètre (24), coopérant avec ledit piston (60) lors de l'actionnement.

8. Dispositif selon la revendication 7, dans lequel ledit piston (60) comporte une lèvre externe (61) pour réaliser, en cours d'actionnement, l'étanchéité avec la partie de plus petit diamètre (24), ladite partie de plus grand diamètre (23) comportant des nervures pour garantir, en position de repos, un passage de fluide entre ledit réservoir (22) et ledit piston (60) vers ladite chambre de dosage (80).

9. Dispositif selon l'une quelconque des revendications précédentes, comportant en outre un insert (45) disposé dans ledit canal d'expulsion (40) pour limiter le volume mort dudit canal d'expulsion (40).

10. Dispositif selon l'une quelconque des revendications précédentes, dans lequel ledit dispositif de distribution comporte en outre un système de génération d'écoulement de gaz comprimé adapté à distribuer un écoulement de gaz comprimé à chaque actionnement.

11. Dispositif selon la revendication 10, dans lequel ledit système de génération d'écoulement de gaz comprimé comprend un autre réservoir (110) contenant une pluralité de doses d'au moins un gaz propulseur pressurisé et/ou liquéfié, une valve doseuse (140) étant montée sur ledit second réservoir (110) pour distribuer une dose de gaz propulseur à chaque actionnement dudit dispositif de distribution.

12. Dispositif selon la revendication 11, dans lequel ladite valve doseuse (140) comportant une soupape (141) déplaçable axialement et qui s'étend à l'intérieur de ladite chambre de dosage (80), de sorte qu'en fin d'actionnement, ledit organe tubulaire axial (50) vient pousser axialement ladite soupape (141) et ainsi actionner la valve (140), provoquant l'expulsion d'un écoulement de gaz comprimé qui va pousser la dose de produit fluide à travers l'orifice de distribution (11) et/ou purger le canal de distribution (40) de tout produit résiduel.

## Patentansprüche

1. Vorrichtung zur Abgabe eines flüssigen Produkts, umfassend:
- einen äußeren Körper (10), der mit einer Abgabeöffnung (11) versehen ist,
- einen inneren Körper (20), der in Bezug auf den äußeren Körper (10) zwischen einer Ruhestellung und einer Betätigungsstellung axial beweglich ist, wobei der innere Körper (20) einen Vorratsbehälter (22) bildet, der das abzugebende flüssige Produkt enthält,
- ein erstes elastisches Element (30), beispielsweise eine Feder, mittels der der innere Körper (20) in Richtung seiner Ruheposition vorgespannt wird,
- ein axiales rohrförmiges Organ (50), das im Inneren des inneren Körpers (20) an dem äußeren Körper (10) befestigt ist und teilweise einen Ausstoßkanal (40) definiert, der in der Abgabeöffnung (11) endet,
- einen Kolben (60), der in dem Vorratsbehälter (22) zwischen einer Ruheposition und einer Abgabeposition verschiebbar um das axiale rohrförmige Organ (50) herum gelagert ist,
- ein zweites elastisches Element (70), beispielsweise eine Feder, mittels der der Kolben (60) in Richtung seiner Ruheposition vorgespannt wird,
wobei die Abgabevorrichtung **dadurch gekennzeichnet ist, dass** sie des Weiteren umfasst:
- eine Dosierkammer (80), die in dem inneren Körper (20) zwischen dem Kolben (60) und dem axialen rohrförmigen Element (50) gebildet ist, wobei die Dosierkammer (80) in der Ruhestellung mit dem Vorratsbehälter (22) verbunden und von dem Ausstoßkanal (40) getrennt ist, und während der Betätigung von dem Vorratsbehälter (22) getrennt und mit dem Ausstoßkanal (40) verbunden ist,
- wobei die Dosierkammer (80) in der aufrechten Gebrauchs- und Lagerposition unter dem Vorratsbehälter (22) angeordnet ist, um sich so im Ruhezustand automatisch durch Schwerkraft mit dem in dem Vorratsbehälter enthaltenen flüssigen Produkt zu füllen.

2. Vorrichtung nach Anspruch 1, bei der der Kolben (60) in seiner Ruhestellung mindestens einen radialen Kanal (52) des axialen rohrförmigen Elements (50) verschließt, um die Dosierkammer (80) vom Ausstoßkanal (40) zu trennen.

3. Vorrichtung nach Anspruch 2, bei der bei Betätigung der Vorrichtung der Kolben (60) axial um das axiale rohrförmige Element (50) herum in seine Abgabeposition verschoben wird, um dadurch die Dosierkammer (80) über den mindestens einen radialen Kanal (52) mit dem Ausstoßkanal (40) zu verbinden.

4. Vorrichtung nach einem der vorhergehenden Ansprüche, bei der sich die Dosierkammer (80) um ein distales Ende (56) des axialen rohrförmigen Elements (50) herum und unter diesem erstreckt.

5. Vorrichtung nach Anspruch 4, bei der der Teil der Dosierkammer (80), der unter dem axialen rohrförmigen Element (50) angeordnet ist, durch eine Vertiefung (81) gebildet wird, die in einem axialen Boden des inneren Körpers (20) ausgebildet ist.

6. Vorrichtung nach einem der vorhergehenden Ansprüche, bei der ein radial äußerer Vorsprung (55) des axialen rohrförmigen Elements (50) im Ruhezustand und bei Betätigung abdichtend mit dem inneren Körper (20) zusammenwirkt, um den Vorratsbehälter (22) von der Atmosphäre zu trennen.

7. Vorrichtung nach einem der vorhergehenden Ansprüche, bei der der Vorratsbehälter (22) einen Teil mit größerem Durchmesser (23), der in der Ruhestellung mit dem Kolben (60) zusammenwirkt, und einen Teil mit kleinerem Durchmesser (24), der bei Betätigung mit dem Kolben (60) zusammenwirkt, aufweist.

8. Vorrichtung nach Anspruch 7, bei der der Kolben (60) eine äußere Lippe (61) aufweist, um während der Betätigung die Abdichtung des Teils mit kleinerem Durchmesser (24) zu bewirken, wobei der Teil mit größerem Durchmesser (23) mit Rippen versehen ist, um in der Ruhestellung einen Flüssigkeitsdurchlass zwischen dem Vorratsbehälter (22) und dem Kolben (60) zu der Dosierkammer (80) hin zu gewährleisten.

9. Vorrichtung nach einem der vorhergehenden Ansprüche, die des Weiteren einen Einsatz (45) umfasst, der in dem Ausstoßkanal (40) angeordnet ist, um das Totvolumen des Ausstoßkanals (40) zu begrenzen.

10. Vorrichtung nach einem der vorhergehenden Ansprüche, bei der die Abgabevorrichtung des Weiteren ein System zur Erzeugung eines Druckgasstroms aufweist, das dazu geeignet ist, bei jeder Betätigung einen Druckgasstrom abzugeben.

11. Vorrichtung nach Anspruch 10, bei der das System zur Erzeugung eines Druckgasstroms einen weiteren Vorratsbehälter (110) umfasst, der eine Vielzahl von Dosen mindestens eines Drucktreibgases und/oder eines verflüssigten Treibgases enthält, wobei ein Dosierventil (140) an dem zweiten Vorratsbehälter (110) angebracht ist, um bei jeder Betätigung der Abgabevorrichtung eine Dosis Treibgas abzugeben.

12. Vorrichtung nach Anspruch 11, bei der das Dosierventil (140) ein Ventil (141) aufweist, das axial verschiebbar ist und sich innerhalb der Dosierkammer (80) erstreckt, so dass bei Ende der Betätigung das axiale röhrenförmige Element (50) das Ventil (141) axial nach unten drückt und dadurch das Ventil (140) betätigt, wodurch ein Druckgasstrom ausgestoßen wird, der die Dosis des flüssigen Produkts durch die Abgabeöffnung (11) hindurch drückt und/oder jegliche Produktrückstände aus dem Abgabekanal (40) entfernt.

## Claims

1. A device for dispensing a fluid product including:
- an outer body (10) provided with a dispensing orifice (11),
- an inner body (20), axially movable relative to said outer body (10) between a rest position and an actuation position, said inner body (20) forming a tank (22) containing the fluid product to be dispensed,
- a first elastic element (30), such as a spring, to bias said inner body (20) towards its rest position,
- an axial tubular member (50) fixed to said outer body (10), inside said inner body (20), and partly defining an expulsion channel (40) ending in said dispensing orifice (11),
- a piston (60) slidably mounted in said tank (22), around said axial tubular member (50), between a rest position and a dispensing position,
- a second elastic element (70), such as a spring, to bias said piston (60) towards its rest position,
the dispensing device being **characterized in that** it further includes:
- a metering chamber (80) defined in said inner body (20) between said piston (60) and said axial tubular member (50), said metering chamber (80) being, in the rest position, connected to said tank (22) and isolated from said expulsion channel (40), and being, during actuation, isolated from said tank (22) and connected to said expulsion channel (40),
- said metering chamber (80) being, in the upright position of use and storage, arranged under said tank (22), so that at rest it is automatically filled by gravity with the fluid product contained in said tank.

2. The device according to claim 1, wherein, in its rest position, said piston (60) closes off at least one radial channel (52) of said axial tubular member (50), to isolate said metering chamber (80) from said expulsion channel (40).

3. The device according to claim 2, wherein, when the device is actuated, said piston (60) is moved axially around said axial tubular member (50) to its dispensing position, thus connecting said metering chamber (80) to said expulsion channel (40) via said at least one radial channel (52).

4. The device according to any one of the preceding claims, wherein said metering chamber (80) extends around and below a distal end (56) of said axial tubular member (50).

5. The device according to claim 4, wherein said part of the metering chamber (80) disposed under said axial tubular member (50) is formed of a well (81) made in an axial bottom of said inner body (20).

6. The device according to any one of the preceding claims, wherein an external radial projection (55) of said axial tubular member (50) cooperates in a sealed manner with said inner body (20), at rest and during actuation, in order to isolate the tank (22) from the atmosphere.

7. The device according to any one of the preceding claims, wherein said tank (22) includes a larger diameter part (23), cooperating with said piston (60) in the rest position, and a smaller diameter part (24), cooperating with said piston (60) during actuation.

8. The device according to claim 7, wherein said piston (60) includes an outer lip (61) to achieve, during actuation, the sealing with the smaller diameter part (24), said larger diameter part (23) including ribs to guarantee, in the rest position, a passage of fluid between said tank (22) and said piston (60) towards said metering chamber (80).

9. The device according to any one of the preceding claims, further including an insert (45) disposed in said expulsion channel (40) to limit the dead volume of said expulsion channel (40).

10. The device according to any one of the preceding claims, wherein said dispensing device further includes a system for generating a flow of compressed gas adapted to dispense a flow of compressed gas upon each actuation.

11. The device according to claim 10, wherein said compressed gas flow generation system comprises another tank (110) containing a plurality of doses of at least one pressurized and/or liquefied propellant gas, a metering valve (140) being mounted on said second tank (110) to dispense a dose of propellant gas each time said dispensing device is actuated.

12. The device according to claim 11, wherein said metering valve (140) including a valve (141) movable axially and which extends inside said metering chamber (80), so that at the end of actuation, said axial tubular member (50) axially pushes said valve (141) and thus actuates the valve (140), causing the expulsion of a flow of compressed gas which will push the dose of fluid product through the dispensing orifice (11) and/or purge the dispensing channel (40) of any residual product.
